# DEMANDE DE BREVET EUROPEEN

(11) **EP 1 076 098 A1**
(43) Date de publication de la demande: **14.02.2001**
(21) Numéro de dépôt: 99410097.2
(22) Date de dépôt: 12.08.1999
(51) Int. Cl.: C12Q 1/18, C12Q 1/04

(54) **Procédé d'assistance à l'interprétation de tests de sensibilité de bactéries à des antibiotiques**

(71) Demandeur: BIOMERIEUX, 69280 Marcy-L'Etoile (FR)
(72) Inventeur: Lazzarini, Annie, 01500 Ambronay (FR); Boeufgras, Jean-Marc, 01150 Vaux en Bugey (FR)
(74) Mandataire: de Beaumont, Michel

(57) **Abrégé**

L'invention concerne un procédé d'assistance à l'interprétation de tests de sensibilité de micro-organismes à des agents antimicrobiens, comprenant les étapes suivantes pour chacun de plusieurs mécanismes de résistance d'un micro-organisme prédéterminé :
mesurer les concentrations minimales inhibitrices (CMI) de plusieurs agents antimicrobiens pour le micro-organisme ;
afficher graphiquement les CMI mesurées dans des zones adjacentes semblables graduées en CMI associées respectivement aux agents antimicrobiens ; et
afficher graphiquement en superposition avec les CMI mesurées des intervalles de CMI statistiques correspondant aux agents antimicrobiens et au mécanisme de résistance.

## Description

La présente invention concerne un procédé d'analyse de résultats de tests de sensibilité de bactéries à des antibiotiques afin d'assister le médecin à prescrire un traitement, et plus spécifiquement un procédé facilitant l'interprétation des résultats de l'analyse.

La société Biomérieux commercialise un appareil sous la dénomination "Vitek 2" mettant en oeuvre un tel procédé d'analyse. Il permet, à partir d'une identification sommaire d'une espèce d'une souche et d'un test d'antibiogramme, de valider le test et d'indiquer le mécanisme de résistance de la souche. Le procédé est décrit plus en détail dans la demande de brevet européen EP-A-0 878 550.

Le procédé mis en oeuvre par cet appareil utilise une base de données répertoriant des agents antimicrobiens et des espèces de micro-organismes avec leurs mécanismes de résistance aux différentes familles d'agents antimicrobiens. Pour chaque agent antimicrobien et chaque mécanisme de résistance, la base de données stocke des paramètres caractéristiques d'une distribution statistique de concentration minimale inhibitrice (CMI).

Ces paramètres sont, par exemple, une moyenne et un écart-type, ou les limites inférieure et supérieure de la distribution et une information sur sa forme, ou encore les effectifs normalisés de chaque classe de valeurs. Cette distribution statistique est le résultat de tests effectués sur un grand nombre d'échantillons d'individus de même espèce et de même mécanisme de résistance, c'est-à-dire sur une population représentative de l'espèce et du mécanisme de résistance.

Pour utiliser l'appareil d'analyse susmentionné, on réalise, à l'aide d'un test bactériologique, une identification plus ou moins précise de l'espèce à laquelle appartient la souche étudiée, par exemple à l'aide de réactifs biochimiques d'identification. En même temps, on réalise un test d'antibiogramme avec un certain nombre d'antibiotiques. Ce test d'antibiogramme fournit une mesure de la CMI de la souche étudiée à différents antibiotiques, ou permet de situer cette CMI dans un intervalle donné.

Une fois que ces tests ont été enregistrés par l'appareil d'analyse, ce dernier extrait de la base de données les distributions de CMI associées aux mécanismes de résistance de l'espèce identifiée et aux antibiotiques testés. Les CMI mesurées, fournies par les antibiogrammes, sont alors comparées aux distributions de CMI extraites pour fournir un indice synthétique reflétant l'adéquation des CMI mesurées pour les antibiotiques testés à chaque mécanisme de résistance.

Si cet indice synthétique a une valeur suffisamment élevée pour l'un des mécanismes de résistance, ce mécanisme de résistance est celui que l'on cherche à identifier et le test est déclaré valide.

Si l'indice synthétique à une valeur trop faible, le test n'est pas valide, et l'appareil d'analyse est capable de proposer une correction pour une ou plusieurs des CMI mesurées, en recherchant une correction optimale selon un certain nombre de critères. On cherche notamment à minimiser le nombre d'antibiotiques corrigés, à minimiser le nombre de corrections à la baisse, à minimiser l'ampleur des corrections, à maximiser le niveau d'adéquation des CMI non corrigées aux mécanismes de résistance utilisés, à maximiser la fréquence avec laquelle ces mécanismes de résistance peuvent être rencontrés.

La plupart du temps, les corrections proposées par l'appareil d'analyse sont fiables. Toutefois, avec les informations actuellement fournies par l'appareil d'analyse au biologiste, celui-ci ne peut pas vérifier de manière simple la proposition de correction. En particulier, il sera difficile pour le biologiste de constater que la correction proposée est erronée et/ou proposer une meilleure correction compte tenu de son expérience.

Par ailleurs, il arrive parfois que l'indice synthétique susmentionné soit suffisamment élevé pour plusieurs mécanismes de résistance. Dans ce cas, l'appareil d'analyse propose le mécanisme pour lequel l'indice est maximal, mais le biologiste pourrait hésiter entre les mécanismes de résistance.

Un objet de la présente invention est de prévoir un procédé d'assistance à l'interprétation des résultats permettant à l'utilisateur de vérifier de manière particulièrement simple les propositions faites par l'appareil d'analyse, notamment de constater des erreurs et proposer des corrections.

Pour atteindre cet objet, la présente invention prévoit un procédé d'assistance à l'interprétation de tests de sensibilité de micro-organismes à des agents antimicrobiens, comprenant les étapes suivantes pour chacun de plusieurs mécanismes de résistance d'un micro-organisme prédéterminé :
mesurer les concentrations minimales inhibitrices (CMI) de plusieurs agents antimicrobiens pour le micro-organisme ;
afficher graphiquement les CMI mesurées dans des zones adjacentes semblables graduées en CMI associées respectivement aux agents antimicrobiens ; et
afficher graphiquement en superposition avec les CMI mesurées des intervalles de CMI statistiques correspondant aux agents antimicrobiens et au mécanisme de résistance.

Les agents antimicrobiens susmentionnés peuvent être des agents antimicrobiens différents appartenant à une même famille d'antibiotiques, des agents antimicrobiens différents appartenant à des familles d'antibiotiques différentes, ou encore des agents antimicrobiens individuellement différents les uns des autres.

Selon un mode de réalisation de la présente invention, les zones adjacentes sont des secteurs de disque, la graduation de CMI étant croissante du centre vers l'extérieur.

Selon un mode de réalisation de la présente invention, le procédé comprend les étapes consistant à marquer visuellement les zones dans lesquelles les CMI mesurées ne recoupent pas les intervalles de CMI statistiques, et à afficher en même temps les résultats pour tous les mécanismes de résistance.

Selon un mode de réalisation de la présente invention, les intervalles de CMI statistiques sont affichés sous forme de barres et les CMI mesurées sous forme de points ou de traits.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
les figures 1A à 1E représentent, pour différents mécanismes de résistance d'une même espèce, des résultats de mesure affichés selon le procédé de l'invention, dans un cas où les mesures sont cohérentes avec la base de données ;
Les figures 2A à 2E représentent le même affichage que les figures 1A à 1E dans un cas où les mesures sont incohérentes avec la base de données.

Selon la présente invention, pour faciliter l'interprétation des résultats de tests fournis par un appareil d'analyse utilisant une base de données (de type Vitek 2 susmentionné ou similaire), on prévoit d'afficher de manière graphique et superposée des informations provenant de la base de données et des informations provenant des tests de sensibilité.

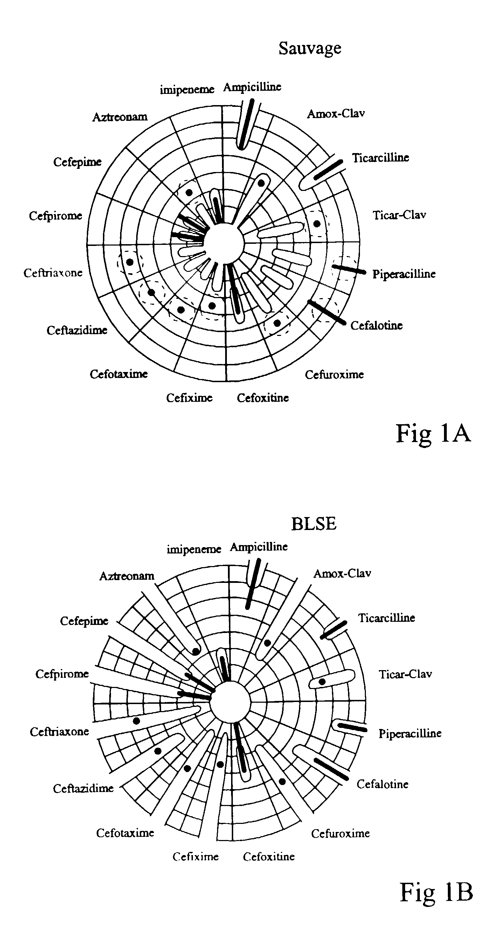

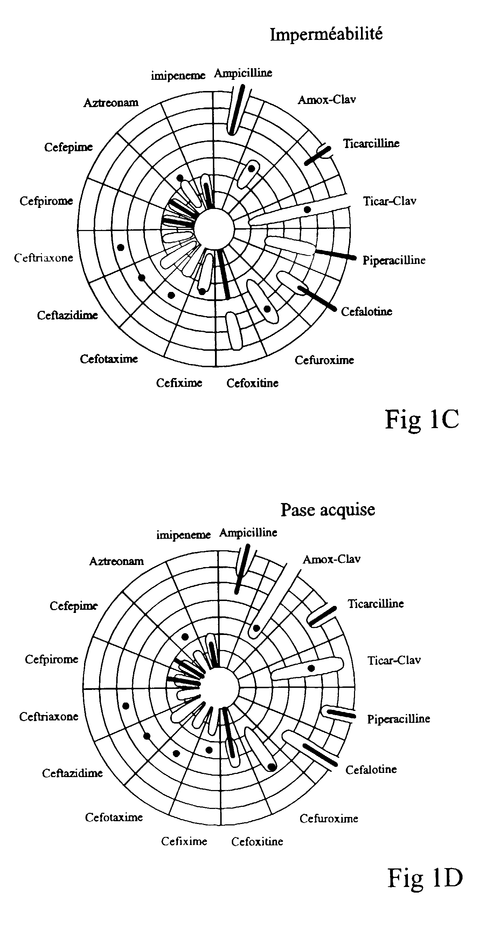

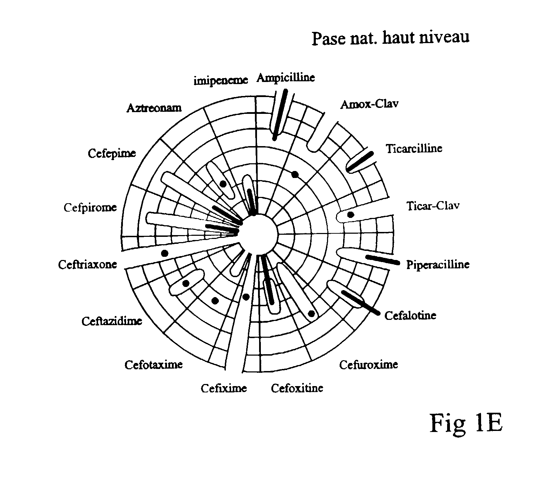

Les figures 1A à 1E représentent les affichages obtenus selon la présente invention pour un premier exemple de test où il n'y a aucune ambiguïté quant à l'identification d'une espèce et son mécanisme de résistance par l'appareil d'analyse.

Dans ce premier exemple, on a sommairement identifié l'espèce comme étant *Klebsiella pneumoniae - ssp pneumoniae* qui peut posséder l'un de cinq mécanismes de résistance à identifier, à savoir sauvage, BLSE (Bétalactamase à Spectre Etendu), Imperméabilité, Pase acquise (pénicillinase), et Pase naturelle haut niveau.

Les CMI mesurées pour différents agents antimicrobiens sont indiquées dans le tableau ci-dessous (les agents antimicrobiens testés seront généralement d'une même famille) :

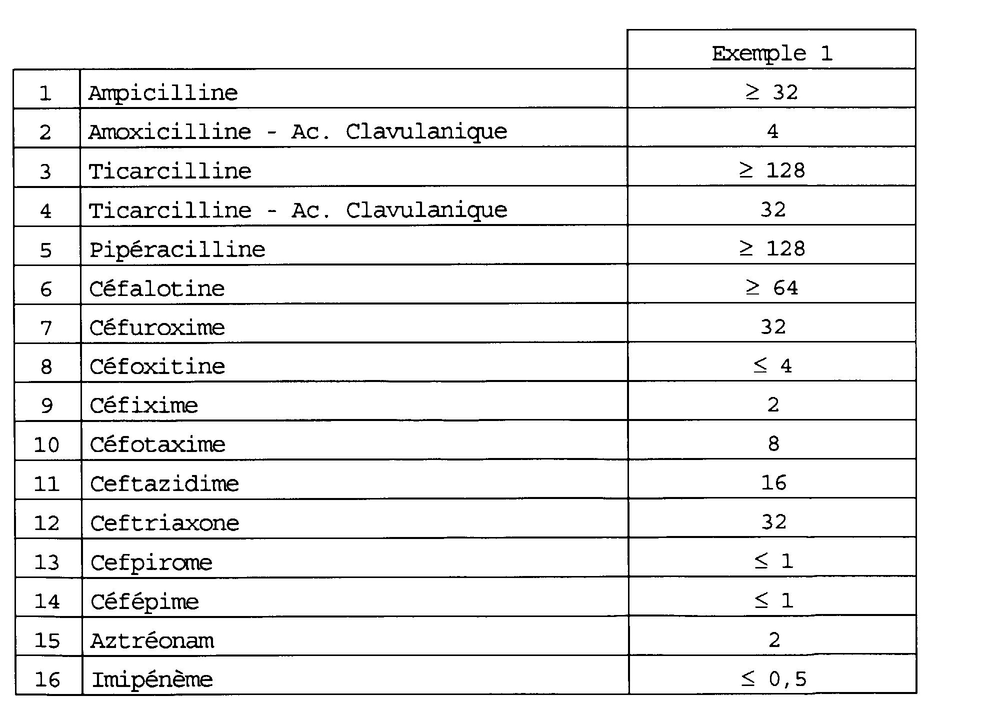

Ces résultats de mesure sont à comparer aux informations correspondantes de la base de données de l'appareil d'analyse. L'appareil Vitek 2 susmentionné stocke des informations sur des distributions statistiques de CMI pour chaque agent antimicrobien à tester et chaque mécanisme de résistance des espèces répertoriées. Pour les besoins de la présente invention, on n'utilise que les intervalles de ces distributions et non les effectifs des classes.

Le tableau ci-dessous indique les intervalles de CMI de la base de données pour les mécanismes de résistance possibles de l'espèce Klebsiella pneumoniae-ssp pneumoniae et les agents antimicrobiens testés du tableau précédent.

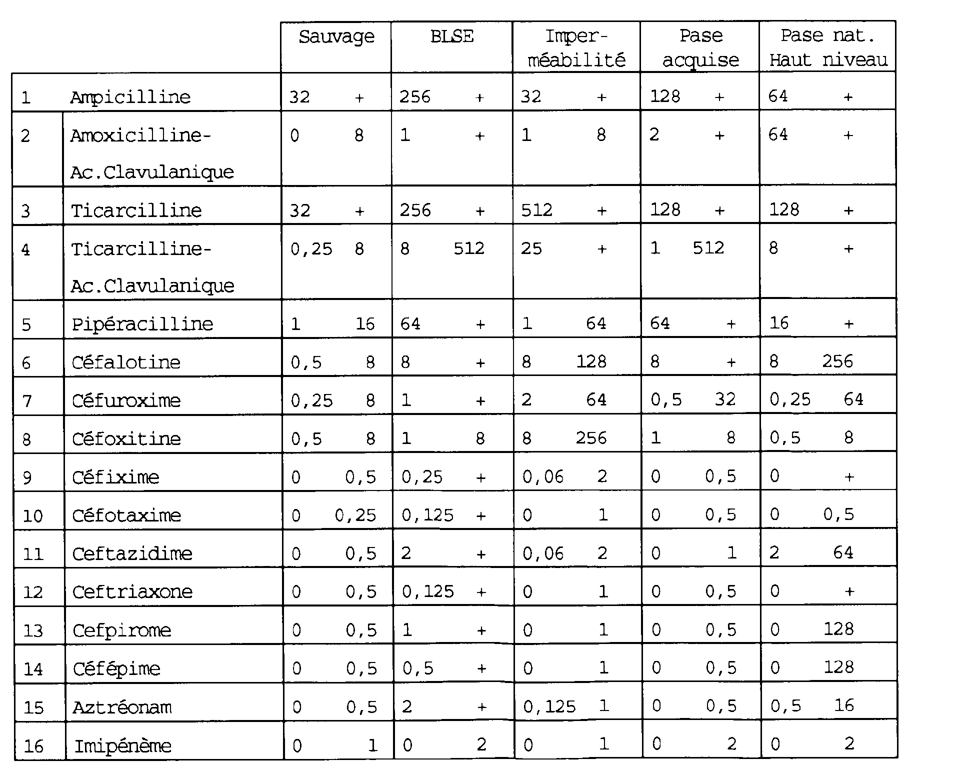

Les figures 1A à 1E correspondent respectivement aux mécanismes de résistance de l'espèce étudiée. Elles illustrent des graphiques représentant les données des tableaux précédents de manière à permettre leur interprétation par l'utilisateur de façon particulièrement simple. Les graphiques correspondant aux différents mécanismes de résistance sont de préférence affichés en même temps devant l'utilisateur.

Chacune des figures représente un disque qui est partagé en secteurs dont chacun correspond à l'un des agents antimicrobiens testés. Le disque est gradué en CMI, les CMI étant croissantes radialement du centre vers l'extérieur. Comme cela est représenté, la graduation peut être marquée par des cercles concentriques, les graduations représentées étant, à titre d'exemple, 0,0625, 0,256, 1, 4, 16, 64, 256 et 1024 en allant du plus petit cercle vers le plus grand.

Dans chaque secteur, on trace la CMI mesurée pour l'agent antimicrobien correspondant. Les CMI mesurées peuvent être sous forme de valeurs isolées ou de plages de valeurs. Lorsqu'il s'agit d'une valeur isolée, celle-ci est tracée sous la forme d'un point distinctement visible. S'il s'agit d'un intervalle, celui-ci est tracé sous la forme d'un trait distinctement visible couvrant l'intervalle. Les CMI mesurées sont reportées à l'identique dans chacun des graphiques des figures 1A à 1E.

En superposition avec ces valeurs mesurées, on affiche également les informations correspondantes contenues dans la base de données. Dans chaque secteur, on affiche l'intervalle de CMI statistique correspondant sous la forme d'une barre oblongue s'étendant sur l'intervalle, les barres étant plus épaisses que les points ou les traits utilisés pour représenter les CMI mesurées. Les couleurs des barres et des traits sont bien entendu différentes pour qu'on puisse les différencier lorsqu'ils se superposent.

La base de données stocke des intervalles de CMI statistiques différents d'un mécanisme de résistance à l'autre pour une espèce donnée. Ainsi, les tracés de ces barres diffèrent d'une figure à l'autre.

Dans le présent exemple, il n'y a pas d'incohérence entre les CMI mesurées et la base de données. L'appareil d'analyse valide le test et indique que le mécanisme de résistance est BLSE. En observant les figures 1A à 1E, l'utilisateur pourra immédiatement vérifier cette conclusion. En effet, le graphique de la figure 1B, correspondant au mécanisme BLSE, est le seul où chacun des traits ou points correspondant aux CMI mesurées se superpose au moins partiellement à une barre représentant un intervalle de CMI statistique de la base de données. Dans tous les autres graphiques, plusieurs points ou traits correspondant aux CMI mesurées sont nettement en dehors des barres correspondantes.

Pour simplifier davantage l'interprétation de ces graphiques, notamment si un grand nombre de graphiques est affiché en même temps, et donc qu'ils sont de petite taille, on peut marquer visuellement chacun des secteurs où la valeur mesurée est en dehors de l'intervalle fourni par la base de données. Un tel marquage consiste, par exemple, à afficher dans les secteurs concernés un point rouge autour des valeurs mesurées, comme cela est indiqué en pointillés à la figure lA. Selon une variante, on marque par un point vert les secteurs où il y a correspondance entre les valeurs mesurées et les valeurs de la base de données.

Dans le cas de ce premier exemple, le procédé selon l'invention permet simplement de confirmer que les résultats fournis par l'appareil d'analyse classique sont cohérents.

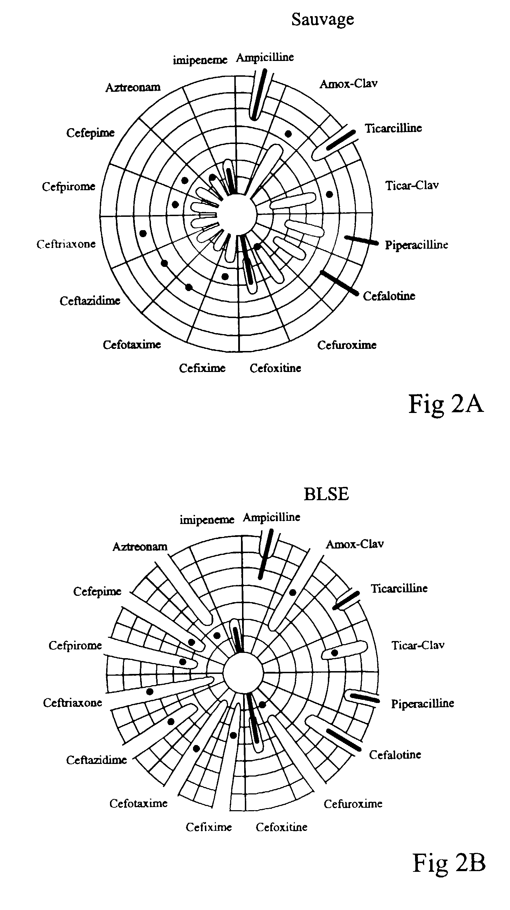

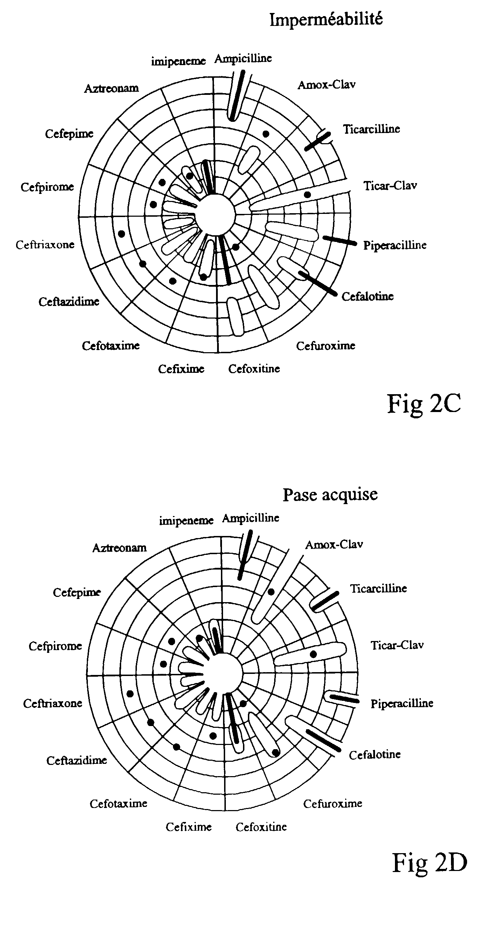

les figures 2A à 2E correspondent à un exemple où l'appareil d'analyse classique indique une incohérence et serait susceptible de proposer une correction erronée. C'est dans ce cas que le procédé de l'invention est particulièrement utile, car il permet au biologiste de déceler immédiatement qu'il y a une incohérence et de voir quelles corrections il faut apporter, si une correction est possible.

Dans ce deuxième exemple, les CMI mesurées sont fournies par le tableau suivant :

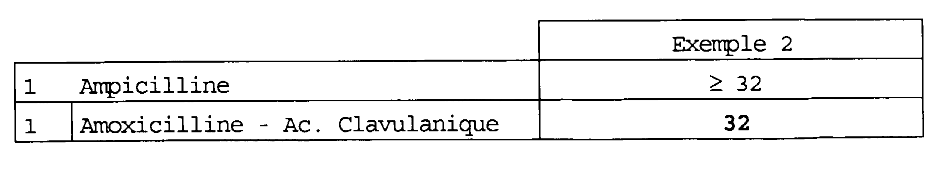

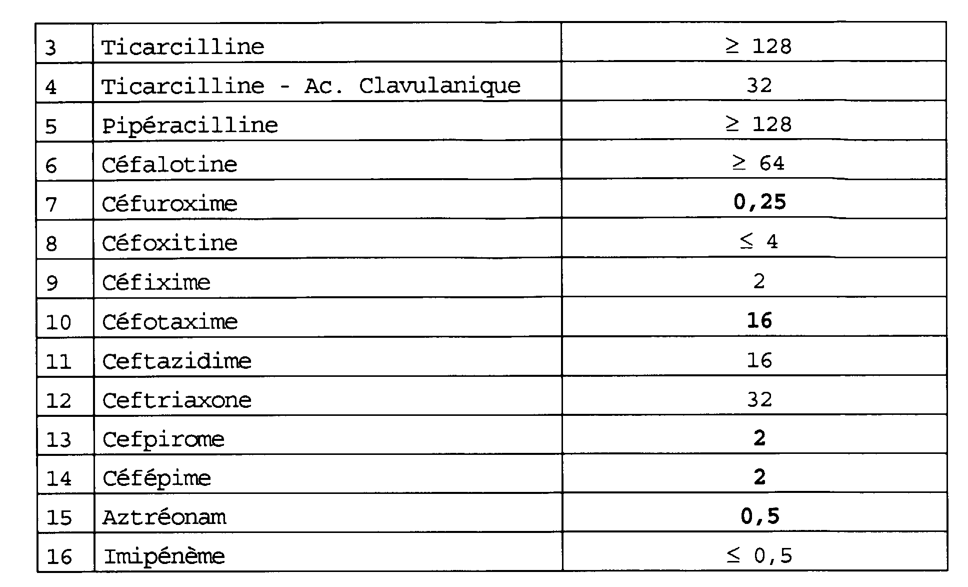

Les valeurs qui diffèrent par rapport à l'exemple 1 sont marquées en gras. Ce sont les CMI mesurées pour l'Amoxicilline-Acide Clavulanique, le Céfuroxime, le Céfotaxime, le Cefpirome, le Céfépime, et l'Aztréonam.

Dans cet exemple, l'appareil d'analyse classique conclut que les résultats sont incohérents et propose probablement une correction de CMI pour le Céfotaxime pour la faire passer de 16 à 0,5. Cette proposition repose sur la reconnaissance phénotypique du mécanisme de résistance Pase naturelle haut niveau, auquel correspond un risque d'échec thérapeutique beaucoup plus limité que pour le mécanisme de résistance BLSE. Les graphiques permettent cependant de mettre en évidence que l'appareil d'analyse a éliminé la possibilité du mécanisme BLSE sur la base de deux antibiotiques, le Céfuroxime et l'Aztréonam, pour lesquels la CMI mesurée apparaissait trop basse, et que la CMI de l'Amoxicilline-Acide Clavulanique est à la limite inférieure des valeurs possibles pour le mécanisme Pase haut niveau.

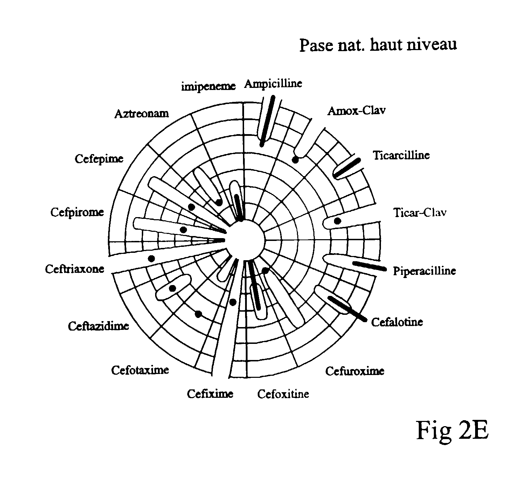

Cette observation conduira probablement le biologiste à refuser la proposition et à refaire l'analyse. Dans l'attente, il n'écartera pas la possibilité d'un mécanisme BLSE s'il doit rendre une réponse de façon urgente. En effet, dans le graphique de la figure 2E, on peut en alternative corriger les CMI mesurées pour l'Aztréonam et le Céfuroxime à la hausse pour que les résultats soient cohérents et que l'on identifie le mécanisme BLSE. Le biologiste pourra décider dans ce cas qu'il est plus sûr de corriger faiblement à la hausse les valeurs pour l'Aztréonam et le Céfuroxime (c'est-à-dire déclarer l'espèce plus résistante à ces deux antibiotiques) que de corriger une seule valeur de CMI à la baisse pour le Céfotaxime (c'est-à-dire déclarer l'espèce plus sensible au Céfotaxime).

De nombreuses variantes et modifications de la présente invention apparaîtront à l'homme du métier. Par exemple, bien que l'on préfère une représentation des graphiques sous forme de disques et de secteurs, la représentation pourrait être linéaire. Les cercles concentriques marquant les graduations de CMI sont purement indicatifs et peuvent être omis.

Les plages de CMI normalisées pour chaque agent antimicrobien et mécanisme de résistance d'une espèce, à savoir les plages où l'on déclare l'espèce "sensible", "intermédiaire", ou "résistante", peuvent être marquées par des couleurs différentes dans les secteurs.

## Revendications

1. Procédé d'assistance à l'interprétation de tests de sensibilité de micro-organismes à des agents antimicrobiens, comprenant les étapes suivantes pour chacun de plusieurs mécanismes de résistance d'un micro-organisme prédéterminé :
• mesurer les concentrations minimales inhibitrices (CMI) de plusieurs agents antimicrobiens pour le micro-organisme ;
• afficher graphiquement les CMI mesurées dans des zones adjacentes semblables graduées en CMI associées respectivement aux agents antimicrobiens ; et
• afficher graphiquement en superposition avec les CMI mesurées des intervalles de CMI statistiques correspondant aux agents antimicrobiens et au mécanisme de résistance.

2. Procédé selon la revendication 1, caractérisé en ce que les zones adjacentes sont des secteurs de disque, la graduation de CMI étant croissante du centre vers l'extérieur.

3. Procédé selon la revendication 1, caractérisé en ce qu'il comprend les étapes consistant à marquer visuellement les zones dans lesquelles les CMI mesurées ne recoupent pas les intervalles de CMI statistiques, et à afficher en même temps les résultats pour tous les mécanismes de résistance.

4. Procédé selon la revendication 1, caractérisé en ce que les intervalles de CMI statistiques sont affichés sous forme de barres et les CMI mesurées sous forme de points ou de traits.
